Europäisches Patentamt

European Patent Office

Office européen des brevets

(19)

(11) Veröffentlichungsnummer : **0 133 195**
**B1**

(12) # EUROPÄISCHE PATENTSCHRIFT

(45) Veröffentlichungstag der Patentschrift :
12.11.86

(51) Int. Cl.⁴ : **A 61 F   5/56**

(21) Anmeldenummer : 84103851.6

(22) Anmeldetag : 06.04.84

(54) Antischnarchkissen.

(30) Priorität : 28.06.83 DEU 8318660

(43) Veröffentlichungstag der Anmeldung :
20.02.85 Patentblatt 85/08

(45) Bekanntmachung des Hinweises auf die Patenterteilung : 12.11.86 Patentblatt 86/46

(84) Benannte Vertragsstaaten :
AT BE CH DE FR GB IT LI LU NL SE

(56) Entgegenhaltungen :
CH-A- 441 616
DE-B- 2 646 643
US-A- 3 480 010
US-A- 4 118 813

(73) Patentinhaber : **Ruf-Technik GmbH**
**Pfützenstrasse 58**
**D-6103 Griesheim (DE)**

(72) Erfinder : **Wentker, Ernst**
**Ginsterweg 1**
**D-5245 Mudersbach (DE)**
Erfinder : **Ruf, Hermann**
**Pfützenstrasse 58**
**D-6103 Griesheim (DE)**

(74) Vertreter : **Zinngrebe, Horst, Dr.rer.nat.**
**Saalbaustrasse 11**
**D-6100 Darmstadt (DE)**

## Beschreibung

Die Erfindung betrifft ein Antischnarchkissen mit einem im Kissen eingebauten Lautsprecher.

Das Antischnarchkissen dient dazu, eine im Schlaf schnarchende Person zu wecken, insbesondere dann, wenn das Schnarchgeräusch eine bestimmte Lautstärke über eine bestimmte Zeitspanne hinweg überschreitet.

Aus der CH-A 441616 ist bereits eine Vorrichtung zum Wecken einer im Schlaf schnarchenden Person bekannt, bei der ein Lautsprecher im Kopfkissen eingebaut ist, wobei der Lautsprecher ein akustisch wahrnehmbares Weckgeräusch erzeugt. Diese Vorrichtung hat jedoch den Nachteil, daß das Mikrophon am Bettrand auf einem Stativ angeordnet und auf den zu überwachenden Schläfer ausgerichtet werden muß. Ferner nimmt die Vorrichtung relativ viel Platz in Anspruch.

Aufgabe der Erfindung ist es, ein praktisch handhabbares Antischnarchkissen mit kleinen Abmessungen zu schaffen, das sowohl zur Stützung des Kopfes als auch als Weckvorrichtung einer darauf liegenden schnarchenden Person dient.

Diese Aufgabe wird erfindungsgemäß durch ein Antischnarchkissen der eingangs genannten Art dadurch gelöst, daß das Kissen aus einem elastisch verformbaren Material besteht, und daß im Kissen eine Weckvorrichtung angeordnet ist, welche mindestens ein Mikrophon, eine mit dem Mikrophon verbundene elektrische Schaltung, mindestens einen an die Schaltung angeschlossenen Lautsprecher und eine netzunabhängige Stromquelle aufweist.

Die mit der Erfindung erzielbaren Vorteile bestehen insbesondere darin, daß keine zusätzlichen Geräte um das Bett herumgestellt oder an diesem angebracht werden müssen. Ferner ist es vorteilhaft, daß das Kissen mit einem auswechselbaren Kissenbezug bezogen werden kann und somit den Reinlichkeitsbedürfnissen entgegenkommt. Ferner wird ein Lerneffekt in der Weise erreicht, daß der Schnarchar in einem psychologischen Lernprozeß die Schlafstörung im Falle seines Schnarchens « autosuggestiv » erwarten muß.

Zweckmäßige Weiterbildungen des Gegenstandes nach Anspruch 1 sind in den Unteransprüchen beschrieben. Ein aus Schaumstoff hergestelltes Antischnarchkissen hat zum einen den Vorteil der einfachen Herstellung, als auch einer gewissen Formstabilität, die für die im Kissen enthaltenen Bauteile erforderlich ist. Die erfindungsgemäße Weckvorrichtung zeichnet sich durch einen einfachen Aufbau aus, wobei besonders die netzunabhängige Stromquelle den Vorteil hat, daß keine Leitungen im Bett verlegt werden müssen und darüber hinaus mit großer Sicherheit Kurzschlüsse vermieden werden. Mit der erfindungsgemäßen elektrischen Schaltung ist es möglich, das akustische Wecksignal in seiner Lautstärke und Tonhöhe einzustellen. Ferner ist es möglich, die Weckreize zu wiederholen und in ihrer Intensität abgestuft auszulösen. Eine am Seitenrand des Antischnarchkissens ausgebildete, der Nackenpartie des Menschen angepaßte Aussparung ermöglicht in einfacher Weise eine Positionierung des Kopfes einer schlafenden Person. Die Bedienungselemente der Weckvorrichtung sind in einer vom Körper der Person entfernt liegenden, am seitlichen Rand ausgeformten Tasche geschützt und leicht zugänglich angeordnet. Vorteilhaft ist insbesondere eine Kontrolleinrichtung, die beispielsweise durch eine aufleuchtende Lampe oder durch ein akustisches Signal (kurzer Pip-ton) anzeigt, ob die Stromquelle funktionstüchtig ist. Die Verwendung einer aufladbaren Stromquelle erspart das Auswechseln von Batterien und ist in einfacher Weise durch einen vorgesehenen Netzanschluß zu bewerkstelligen. Die symmetrische Anordnung von Lautsprechern im oberen Abschnitt des Antischnarchkissens und von Mikrophonen im unteren Abschnitt des Kissens ergeben eine gute Verteilung des Wecksignals und berücksichtigen ferner die verschiedenen Stellungen des Kopfes auf dem Kissen. Die Verlegung der Leitungen zwischen den Mikrophonen und der elektrischen Schaltung und von dieser zu den Lautsprechern vermeidet ein Verrutschen der Leitungen und dadurch bedingte Störungen durch Wackelkontakte. Die Befestigung der Platten aneinander durch einen Klebstoff ermöglicht eine einfache Herstellung. Eine weitere Vereinfachung der Herstellung und eine Erhöhung der Formstabilität wird dadurch erreicht, daß das Schaumstoffteil vom oberen Rand bis zu den im unteren Abschnitt liegenden Öffnungen für die Mikrophone geteilt ist und der restliche Teil des Kissens eine Einheit bildet.

Ausführungsformen der Erfindung werden in den Zeichnungen beispielshalber beschrieben. Dabei zeigen :

Figur 1 eine Ansicht von oben eines erfindungsgemäßen Antischnarchkissens ;

Figur 2a eine Ansicht von der Seite längs des Schnittes I-I des in der Figur 1 gezeigten Antischnarchkissens ;

Figur 2b eine Ansicht von der Seite längs des Schnittes I-I einer weiteren Ausführungsform des erfindungsgemäßen Antischnarchkissens ;

Figur 3a eine Ansicht von der Seite, die das im Antischnarchkissen befestigte Gehäuse zeigt ;

Figur 3b eine Ansicht in das geöffnete Gehäuse ;

Figur 4 eine schematische Darstellung der im erfindungsgemäßen Antischnarchkissen verwendeten elektrischen Schaltung.

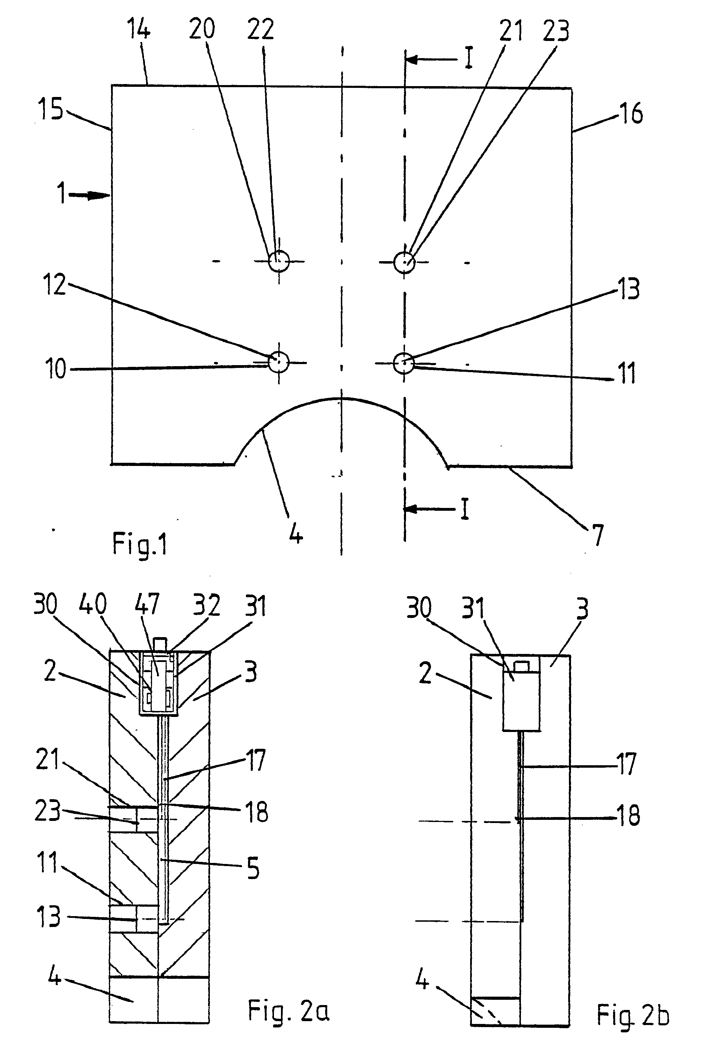

Die Figur 1 zeigt das erfindungsgemäße Antischnarchkissen 1 in einer Ansicht von oben, aus der die rechteckige Form des Antischnarchkissens 1, das im folgenden kurz « Kissen » genannt wird, hervorgeht. Links und rechts der Symmetrielinie sind Öffnungen 10, 11, 20 und 21 abgebildet, wobei in den Öffungen 20 und 21 Lautsprecher 22 und 23 angeordnet sind und der Abstand zwischen den Lausprechern ungefähr der Breite eines Kopfes entspricht. Die Lautsprecher 22 und 23 können den im Telefon verwendeten Lautsprechern entsprechen. In den Öffungen 10 und 11 sind Mikrophone 12 und 13 angeordnet, die den im Telefon verwendeten Mikrophonen entsprechen können. Der Abstand von den auf einer Linie liegenden Lautsprechern 22 und 23 zu den dazu parallel angeordneten Mikrophonen 12 und 13 entspricht ungefähr dem Abstand zwischen dem Ohr und der Kinnpartie eines Menschen. Am unteren Seitenrand 7 ist symmetrisch in der Mitte eine Aussparung 4 für den Kopf vorgesehen, die der Nackenpartie eines Menschen angepaßt ist. In einer bevorzugten Ausführungsform beträgt die Seitenlänge 7, 14 ungefähr 55 cm und die Seitenlänge 15 und 16 ungefähr 45 cm. Die Dicke dieses Kissens liegt bei ungefähr 6 cm. Wie aus den Figuren 2 a) und 2 b) hervorgeht, besteht das Kissen 1 aus zwei aneinander befestigten Schaumstoffplatten 2 und 3, wobei es jedoch auch möglich ist, daß das Kissen z. B. von der Seite 14 bis ungefähr in die Höhe der Öffungen 10 und 11 geteilt ist und der restliche Abschnitt bis zur Seite 7 ein zusammenhängendes ganzes Teil bildet. Ferner kann die Aussparung 4 längs der ganzen Dicke des Kissens 1 ausgebildet sein, wie dies in Figur 2 a) gezeigt ist. Ferner ist es möglich, daß die Aussparung 4 auch einen kugelförmigen oder treppenförmigen Querschnitt aufweist, wie dies in Figur 2 b) gezeigt ist. Ferner zeigt die Figur 2 a) die Anordnung eines Mikrophons 13 und eines Lautsprechers 23 die über Leitungen 17 und 18 mit einer in einer quaderförmigen Tasche 30 angeordneten Platine 47 verbunden sind, wobei die Platine 47 eine elektrische Schaltung 40 trägt, die in der aus Gußharz bestehenden Platine 47 befestigt ist. Die Leitungen 17 und 18 werden in dieser Ausführungsform in Nuten oder Schlitzen 5 geführt, so daß eine sichere Führung der Leitungen gewährleistet ist und Wackelkontakte durch Verschieben der Leitungen vermieden werden. Die Aussparung 30 kann dadurch gebildet werden, daß sowohl in beiden Schaumstoffplatten 2 und 3 jeweils eine quaderförmige Aussparung ausgeformt ist, oder daß bei entsprechender Dicke des einzusetzenden Gehäuses 31 nur in einer Platte eine quaderförmige Aussparung nötig ist. Das quaderförmige Gehäuse 31, das in dieser Ausführungsform mit einem angelenkten Deckel 32 versehen ist, enthält neben der die elektronische Schaltung 40 tragenden Platine 47 ferner die erforderliche Stomquelle 50. In der Regel Wird diese Stromquelle 50 eine handelsübliche austauschbare Batterie sein, jedoch ist es auch möglich, eine aufladbare Stromquelle, beispielsweise einen Akkumulator zu verwenden. Wie aus der Figur 2 b) hervorgeht, können die Leitungen 17 und 18 auch im Zwischenraum zwischen den Schaumstoffplatten 2 und 3 verlegt werden.

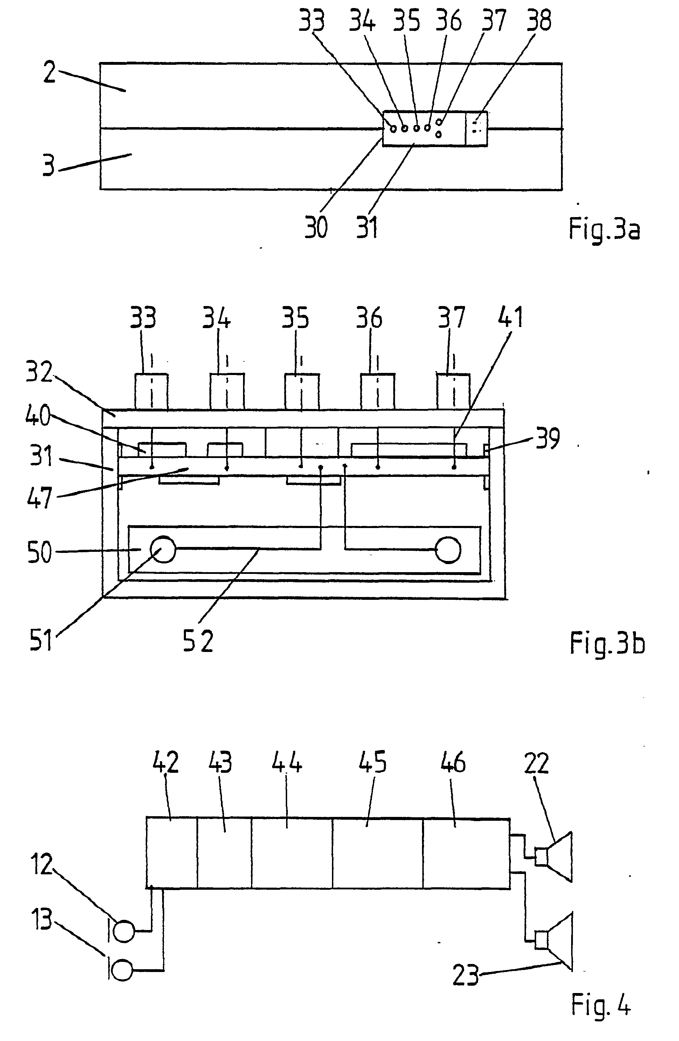

Die Figur 3 a) zeigt eine Seitenansicht des Kissens, bei der die Bedienungselemente, die im Deckel 32 des Gehäuses 31 angeordnet sind, gezeigt sind. Zweckmäßigerweise ist das Gehäuse 31 am Rand der Seiten 14, 15 und 16 angeordnet, so daß eine für die schlafende Person unangenehme Störung durch das Fühlen eines harten Gegenstandes vermieden ist. Wie aus den Figuren 2 a) und 2 b) hervorgeht, können die Bedienungselemente zum einen aus dem Kissen herausragen oder zum anderen durch den umgebenden Schaumstoff geschützt vertieft angeordnet sein. Die Anordnung der Stromquelle 50 und der Platine 47 kann sowohl nebeneinander, wie dies in der Figur 3 a) gezeigt ist, oder aber gegenüber erfolgen, wie dies in der Figur 3 b) abgebildet ist, abhängig von der Dicke der verwendeten Bauteile. Auf dem Deckel 32 sind folgende Bedienungselemente angeordnet : Ein Ein/Aus-Schalter 33, ein Regler 34 für die Lautstärke, ein Regler 35 für die Tonhöhe, ein Regler 36 für das Zeitglied, eine Kontrolleinrichtung 37 für die Stromquelle 50, die anzeigt, ob die Stromquelle funktionstüchtig ist oder wieder aufgeladen oder ausgetauscht werden muß. Ein Netzanschluß 38 ist für die Ausführungsform vorgesehen, die eine aufladbare Stromquelle 50 aufweist.

In der Figur 4 ist eine schematische Darstellung der elektrischen Schaltung 40 abgebildet, an die zwei Mikrophone 12 und 13 und zwei Lautsprecher 22 und 23 angeschlossen sind. In der einfachsten Ausführungsform ist es möglich, nur einen Lautsprecher und ein Mikrophon an die elektrische Schaltung anzuschließen. Ferner ist auch möglich, mehr als zwei Lautsprecher und zwei Mikrophone zu verwenden. Die elektrische Schaltung 40 besteht aus einem Vorverstärker 42, einem Regelglied für die Ansprechempfindlichkeit, einem Zeitglied 44, d. h. einer Kippstufe, einem Oszillator 45, der einen Endverstärker 46 speist. Der Oszillator 45 kann so ausgebildet sein, daß die Lautstärke und die Tonhöhe einstellbar sind. Die Elemente sind zweckmäßigerweise in Gußharz zu einer Platine vergossen.

## Patentansprüche

1. Antischnarchkissen mit einem im Kissen eingebauten Lautsprecher, dadurch gekennzeichnet, daß das Kissen (1) aus einem elastisch verformbaren Material besteht, und daß im Kissen (1) eine Weckvorrichtung (6) angeordnet ist, welche mindestens ein Mikrophon (12, 13), eine mit dem Mikrophon verbundene elektrische Schaltung (40), mindestens einen an die Schaltung (40) angeschlossenen Lautsprecher (22, 23) und eine netzunabhängige Stromquelle (50) aufweist.

2. Antischnarchkissen nach Anspruch 1, dadurch gekennzeichnet, daß das Kissenmaterial ein Schaumstoff ist.

3. Antischnarchkissen nach Anspruch 1 oder 2, dadurch gekennzeichnet, daß die Schaltung (40) aus folgenden Teilen besteht : Einem Vorverstärker (42), einem Regelglied (43) für die Ansprechempfindlichkeit, einem Zeitglied (44), einem Oszillator (45) und einem Endverstärker (46).

4. Antischnarchkissen nach Anspruch 3, dadurch gekennzeichnet, daß der Oszillator (45) in der Lautstärke und der Tonhöhe einstellbar ist.

5. Antischnarchkissen nach einem der vorhergehenden Ansprüche, dadurch gekennzeichnet, daß die elektrische Schaltung (40) in Gußharz eingegossen ist.

6. Antischnarchkissen nach einem der vorhergehenden Ansprüche, dadurch gekennzeichnet, daß das Kissen (1) aus zwei rechteckigen Schaumstoffplatten (2, 3) besteht, daß mindestens in der obenliegenden Schaumstoffplatte an einem Seitenrand (7) eine der Nakkenpartie des Menschen angepaßte Aussparung (4) ausgeformt ist, und daß mindestens in einer der Platten (2, 3) auf der Innenseite am Rand der Seiten (14, 15, 16) eine quaderförmige Tasche (30) ausgebildet ist.

7. Antischnarchkissen nach Anspruch 6, dadurch gekennzeichnet, daß in der durch die quaderförmige Tasche (30) oder Taschen (30) gebildete Aussparung ein stabiles Gehäuse (31) mit einem angelenkten Deckel (32) befestigt ist.

8. Antischnarchkissen nach Anspruch 7, dadurch gekennzeichnet, daß der Deckel (32) einen Ein/Ausschalter (33), einen Regler (34) für die Lautstärke, einen Regler (35) für die Tonhöhe, einen Regler (36) für das Zeitglied, eine Kontrolleinrichtung (37) für die Stromquelle (50) aufweist.

9. Antischnarchkissen nach einem der vorhergehenden Ansprüche, dadurch gekennzeichnet, daß die Stromquelle (50) mindestens eine austauschbare Batterie (53) ist.

10. Antischnarchkissen nach den Ansprüchen 1 bis 8, dadurch gekennzeichnet, daß die Stromquelle (50) wieder aufladbar ist.

11. Antischnarchkissen nach Anspruch 6, dadurch gekennzeichnet, daß im unteren Abschnitt der Schaumstoffplatte (2) symmetrisch zur Mittellinie links und rechts mindestens je eine Öffnung (10, 11) ausgeformt ist, daß in jeder Öffnung (10, 11) ein Mikrophon (12, 13) angeordnet ist, und daß im oberen Abschnitt der Platte (2) symmetrisch zur Mittellinie und von den Öffnungen (10, 11) beabstandet mindestens je eine Öffnung (20, 21) ausgeformt ist, und daß in den Öffnungen (20, 21) je ein Lautsprecher (22, 23) angeordnet ist.

12. Antischnarchkissen nach einem der vorhergehenden Ansprüche, dadurch gekennzeichnet, daß die Leitungen (17) der Mikrophone (12, 13) zur elektrischen Schaltung (40) und die Leitungen (18) von der elektrischen Schaltung (40) zu den Lautsprechern (22, 23) in, in den Platten (2, 3) ausgebildeten, Nuten oder Schlitzen (5) verlaufen.

13. Antischnarchkissen nach einem der vorhergehenden Ansprüche, dadurch gekennzeichnet, daß die Platten (2, 3) miteinander verklebt sind.

14. Antischnarchkissen nach einem der vorhergehenden Ansprüche, dadurch gekennzeichnet, daß die Platten (2, 3) vom unteren Seitenrand (7) bis ungefähr zu den Öffnungen (10, 11) einstückig ausgebildet sind.

**Claims**

1. Antisnoring pillow with a loudspeaker built into the pillow characterized in that the pillow (1) consists of an elastically-deformable material, and that a waking device (6) is arranged within the pillow (1), which includes at least a microphone (12, 13), an electric circuit (40) connected to the microphone, at least one loudspeaker (22, 23) connected to the circuit (40) and a source (50) of current independent of the network.

2. Antisnoring pillow according to claim 1 characterized in that the material of the pillow is a foam plastic.

3. Antisnoring pillow according to claim 1 or 2 characterized in that the circuit (40) comprises the following parts : a preamplifier (42), a regulating member (43) for the response sensitivity, a time member (44), an oscillator (45) and a terminal amplifier (46).

4. Antisnoring pillow according to claim 3 characterized in that the oscillator (45) is adjustable in the volume of sound and the pitch of the tone.

5. Antisnoring pillow according to anyone of the preceding claims characterized in that the electric circuit (40) is embedded within cast resin.

6. Antisnoring pillow according to anyone of the preceding claims characterized in that the pillow (1) consists of two rectangular plates (2, 3) of foam plastic, that an indentation (4) is formed at least in the top foam plastic plate at a lateral margin (7) in a manner adapted to the neck part of the human being and that a square-shaped pocket (30) is formed at least in one of the plates (2, 3) at the inner side of the margin of the sides (14, 15, 16).

7. Antisnoring pillow according to claim 6 characterized in that a stable housing (31) having an articulated lid (32) is mounted within the indentation formed by the square-shaped pocket (30) or pockets (30).

8. Antisnoring pillow according to claim 7 characterized in that the lid (32) has an on/off switch (33), a regulator (34) for the volume of sound, a regulator (35) for the pitch of tone, a regulator (36) for the time member, and a control arrangement (37) for the source (50) of current.

9. Antisnoring pillow according to anyone of the preceding claims characterized in that the source (50) of current is at least an exchangeable battery (53).

10. Antisnoring pillow according to claim 1 through 8 characterized in that the source (50) of current can be recharged.

11. Antisnoring pillow according to claim 6 characterized in that in the lower section of the foam plastic plate (2), symmetrical to the center line, on the left and on the right, always at least one opening (10, 11) is formed, that in everyone of the openings (10, 11) a microphone (12, 13) is arranged and that in the upper section of the plate (2), symmetrical to the center line and at a distance to the openings (10, 11) at least one opening (20, 21) is formed and that in everyone of the openings (20, 21) a loudspeaker (22, 23) is arranged.

12. Antisnoring pillow according to anyone of the preceding claims characterized in that the lines (17) of the microphones (12, 13) run to the electric circuit (40) and that the lines (18) from the electric circuit (40) run to the loudspeakers (22, 23) within grooves or slits (5) formed within the plates (2, 3).

13. Antisnoring pillow according to anyone of the preceding claims characterized in that the plates (2, 3) are glued together.

14. Antisnoring pillow according to anyone of the preceding claims characterized in that the plates (2, 3) are formed in one piece from the lower edge (7) up to about the openings (10, 11).

**Revendications**

1. Oreiller anti-ronflement avec haut-parleur incorporé, caractérisé en ce que l'oreiller (1) est réalisé dans un matériau élastiquement déformable, et en ce que, dans l'oreiller (1), est prévu un dispositif de réveil (6) comprenant au moins un microphone (12, 13), un circuit électrique (40) relié au microphone, au moins un haut-parleur (22, 23) relié au circuit (40), et une source de courant autonome (50).

2. Oreiller anti-ronflement conforme à la revendication 1, caractérisé en ce que l'oreiller est réalisé dans une matière alvéolaire.

3. Oreiller anti-ronflement conforme à la revendication 1 ou 2, caractérisé en ce que le circuit (40) se compose des éléments suivants : un préamplificateur (42), un élément de réglage (43) de la sensibilité de réponse, un élément de temporisation (44), un oscillateur (45) et un amplificateur terminal (46).

4. Oreiller anti-ronflement conforme à la revendication 3, caractérisé en ce que l'intensité sonore et la tonalité de l'oscillateur (45) sont réglables.

5. Oreiller anti-ronflement conforme à l'une quelconque des revendications précédentes, caractérisé en ce que le circuit électrique (40) est scellé dans de la résine moulée.

6. Oreiller anti-ronflement conforme à l'une quelconque des revendications précédentes, caractérisé en ce que l'oreiller (1) est composé de deux plaques rectangulaires de matière alvéolaire (31, 32), en ce qu'un évidement (4) adapté à la forme de la nuque est ménagé sur un bord latéral (7) au moins dans la plaque supérieure de matière alvéolaire, et en ce qu'une poche en forme de parallélépipède rectangle (30) est prévue dans au moins une des plaques (2, 3), sur la partie intérieure, sur le bord des côtés (14, 15, 16).

7. Oreiller anti-ronflement conforme à la revendication 6, caractérisé en ce qu'un boîtier stable (31) avec couvercle articulé (32) est fixé dans l'évidement formé par la ou les poches en forme de parallélépipède rectangle (30).

8. Oreiller anti-ronflement conforme à la revendication 7, caractérisé en ce que le couvercle (32) est muni d'un interrupteur marche-arrêt (33), d'un bouton de réglage d'intensité sonore (34), d'un bouton de réglage de tonalité (35), d'un bouton de réglage (36) de l'élément de temporisation, et d'un système de contrôle (37) de la source de courant (50).

9. Oreiller anti-ronflement conforme à l'une quelconque des revendications précédentes, caractérisé en ce que la source de courant (50) est au moins une pile rechargeable (53).

10. Oreiller anti-ronflement conforme aux revendications 1 à 8, caractérisé en ce que la source de courant (50) est rechargeable.

11. Oreiller anti-ronflement conforme à l'une quelconque des revendications précédentes, caractérisé en ce que, dans la partie inférieure de la plaque de matière alvéolaire, une ouverture (10, 11) est ménagée à gauche et à droite symétriquement à l'axe médian, en ce qu'un microphone (12, 13) est logé dans chaque ouverture, en ce que, dans la partie supérieure de la plaque (2), est prévue au moins une ouverture (20, 21) symétrique à l'axe médian et éloignée des ouvertures (10, 11), et en ce qu'un haut-parleur (22, 23) est logé dans chacune des ouvertures (20, 21).

12. Oreiller anti-ronflement conforme à l'une quelconque des revendications précédentes, caractérisé en ce que les fils (17) des microphones (12, 13) menant au circuit électrique (40) et les fils (18) menant du circuit électrique (40) aux haut-parleurs (22, 23) passent dans des rainures ou fentes (5) pratiquées dans les plaques (2, 3).

13. Oreiller anti-ronflement conforme à l'une quelconque des revendications précédentes, caractérisé en ce que les plaques (2, 3) sont collées ensemble.

14. Oreiller anti-ronflement conforme à l'une quelconque des revendications précédentes, caractérisé en ce que les plaques (2, 3) sont réalisées d'une seule pièce depuis le bord latéral inférieur (7) jusqu'à hauteur des ouvertures (10, 11).

Fig.1

Fig. 2a

Fig. 2b

Fig.3a

Fig.3b

Fig. 4